# FASCICULE DE BREVET EUROPEEN

(11) **EP 4 291 510 B1**
(45) Date de publication et mention de la délivrance du brevet: **18.09.2024**
(21) Numéro de dépôt: 22839684.2
(22) Date de dépôt: 15.12.2022
(51) Int. Cl.: A61F 9/00

(54) **PROCÉDÉ DE FABRICATION D'UN RÉSERVOIR DE FLUIDE DE TRAITEMENT OPHTALMIQUE, RÉSERVOIR DE FLUIDE DE TRAITEMENT OPHTALMIQUE ET DISPOSITIF DE PRÉPARATION EXTEMPORANÉE D'UNE QUANTITÉ D'UN FLUIDE DE TRAITEMENT OPHTALMIQUE**
VERFAHREN ZUR HERSTELLUNG EINES OPHTHALMISCHEN BEHANDLUNGSFLÜSSIGKEITSRESERVOIRS, BEHANDLUNGSFLÜSSIGKEITSRESERVOIR, VORRICHTUNG ZUR EX-TEMPORANEN ZUBEREITUNG EINER BEHANDLUNGSFLÜSSIGKEIT
METHOD FOR MANUFACTURING AN OPHTHALMIC TREATMENT FLUID RESERVOIR, OPHTHALMIC TREATMENT FLUID RESERVOIR, DEVICE FOR EXTEMPORANEOUSLY PREPARING AN OPHTHALMIC TREATMENT FLUID

(30) Priorité: 15.12.2021 EP 21214614
(43) Date de publication de la demande: 20.12.2023
(73) Titulaire: Arcadophta, 31100 Toulouse (FR)
(72) Inventeur: REBOUL, Gérard, 31100 Toulouse (FR)
(74) Mandataire: Gevers Patents
(86) Numéro de dépôt international: PCT/EP2022/086214
(87) Numéro de publication internationale: WO 2023/111212

(56) Documents cités:
- EP-B2- 1 589 926
- IMRAN KHAN: "Filling of Aerosol", 29 July 2017 (2017-07-29), XP002808863, Retrieved from the Internet <URL:https://www.slideshare.net/imrankhan2540/filling-of-aerosol> [retrieved on 20230316]

## Description

### Domaine technique

La présente invention concerne un procédé de fabrication d'un réservoir de fluide de traitement ophtalmique, ainsi qu'un tel réservoir.

### Art antérieur

Certaines interventions chirurgicales ophtalmiques nécessite l'administration de fluide de traitement ophtalmique à un patient. Par exemple, lors du traitement d'un décollement de la rétine par tamponnement, après replacement de la rétine, un gaz fluoré est injecté dans la cavité oculaire du patient dans le but de maintenir la rétine en place pendant sa cicatrisation. Pour des raisons sanitaires, le fluide de traitement ophtalmique doit être le plus pur et le plus stérile possible lorsqu'il est prélevé d'un contenant par le chirurgien ou un de ses assistants préalablement à son dosage éventuel et à son administration au patient.

Jusqu'au début des années 2000, le prélèvement d'un tel fluide se faisait par prélèvement à partir de bombonne de fluide de grand volume, ce qui causait des difficultés tant pour obtenir une quantité appropriée de fluide que pour respecter les conditions sanitaires exigées dans un contexte chirurgical.

Le brevet EP1589926B2 a apporté une solution avantageuse à ce problème en fournissant un dispositif de préparation extemporanée d'une quantité de fluide de traitement ophtalmique stérile, comprenant un réservoir de fluide sous pression adapté pour libérer une quantité adaptée de fluide dans une seringue, et ce dans des conditions stériles, sans contaminer le fluide. Le réservoir de fluide comprend une cartouche rigide et une valve à poche fixée sur la cartouche. Le fluide est enfermé dans la poche portée par la valve et maintenu sous pression par un gaz pousseur comprimé, généralement de l'air comprimé, présent dans la cartouche, autour la poche.

Juste après la fabrication d'un tel réservoir, la poche comprend typiquement entre 95 et 100% en volume de fluide de traitement ophtalmique sous pression. Après fabrication, le réservoir est en général stocké pendant plusieurs mois, voire jusqu'à plusieurs années, avant d'être utilisé. Malgré la structure particulièrement protectrice du réservoir, il a été constaté par l'inventeur que la proportion en fluide libérable par le réservoir dans la seringue diminuait au fil du temps, de l'ordre de 6 à 10% en volume sur trois ans. Ceci est problématique au regard de l'intégrité du contenu de la poche mais aussi pour des raisons chirurgicales étant donné que la proportion en fluide prélevé du réservoir est incertaine en fonction du temps.

Par ailleurs, dans le contexte technique distinct d'un aérosol consistant en une cartouche enfermant un mélange de gaz propulseur et de produit à délivrer, le document « Filling of Aérosol » de Imran Khan, dont le dépôt en ligne est daté du 29 juillet 2017 sur https://www.slideshare.net/imrankhan2540/filling-of-aerosol, il est évoqué que l'air présent dans la cartouche peut être évacué avant ou durant son remplissage.

### Exposé de l'invention

La présente invention concerne un procédé de fabrication d'un réservoir d'un fluide de traitement ophtalmique selon la revendication 1, un réservoir de fluide de traitement ophtalmique selon la revendication 9, un dispositif de préparation extemporanée d'un fluide de traitement ophtalmique selon la revendication 12.

Un objet de la présente invention est de fournir un procédé pour fabriquer un tel réservoir, de sorte qu'il soit davantage fiable.

À cet effet, l'invention propose un procédé de fabrication d'un réservoir d'un premier fluide de traitement ophtalmique comprenant les étapes suivantes (et de préférence dans cet ordre) :
(i) fournir et placer dans une enceinte close :
   - une cartouche rigide comprenant une ouverture,
   - une valve à poche comprenant une poche portée par une valve prolongée d'une paroi d'obturation,
(ii) injecter un fluide intermédiaire sous forme comprimée dans la cartouche via l'ouverture, le fluide intermédiaire comprenant un deuxième fluide de traitement ophtalmique ;
(iii) fixer la paroi d'obturation sur la cartouche, au niveau de l'ouverture, de façon hermétique, et de sorte que la poche soit enfermée dans la cartouche ;
(iv) injecter le premier fluide dans la poche via la valve,
de sorte qu'il soit maintenu sous pression dans la poche par le fluide intermédiaire présent dans la cartouche autour de la poche.

Dans le cadre de l'invention, l'étape (ii) comprend en outre les sous-étapes :
(ii.1) extraire de l'air de la cartouche ;
(ii.2) injecter le deuxième fluide sous forme comprimée dans la cartouche.

Ce procédé permet d'obtenir un tel réservoir qui soit plus fiable, notamment dont le contenu de la poche est plus stable au fil du temps.

En effet, contre toute attente, il a été découvert que la cause principale de la diminution en proportion de fluide libérable par un tel réservoir dans une seringue selon le brevet EP1589926B2 est dû à un échange fluidique par les parois de la poche et/ou la cartouche, et particulièrement par la pénétration du gaz pousseur, typiquement de l'air comprimé, en faible quantité, à travers la paroi de la poche, malgré que celle-ci soit imperméable. En effet, il a été découvert dans ce cadre que les milieux internes et externes de part et d'autre du film complexe formant la poche ont tendance s'équilibrer, de sorte que si l'un d'eux contient principalement de l'air comprimé, la proportion en fluide de traitement ophtalmique dans l'autre milieu va progressivement diminuer.

Le procédé selon l'invention propose avantageusement d'injecter du fluide intermédiaire comprenant un deuxième fluide de traitement ophtalmique dans la cartouche, au sein d'une enceinte close, et ce préalablement à la fixation et au remplissage de la valve à poche. L'usage du deuxième fluide permet en particulier de maintenir une bonne proportion totale de fluides de traitement ophtalmologique dans la poche au fil du temps. En effet, même si des échanges fluidiques ont lieu de part et d'autre de la poche, le fait que le fluide intermédiaire comprenne de ce deuxième fluide permet de limiter la quantité de fluide non approprié, tel que de l'air, qui pénétrerait dans la poche, et se retrouverait par la suite en proportion trop grande au sein de la quantité individuelle de fluide prélevée dans la seringue. En particulier, avantageusement, plus le fluide intermédiaire comprend du deuxième fluide, et plus la proportion totale de (premier et deuxième) fluides de traitement ophtalmologique dans la poche est stable au fil du temps.

De façon intuitive, cet effet peut également être expliqué par le fait que le deuxième fluide comprend des molécules plus grosses que celles de l'air, et que ces molécules ont davantage de difficulté à traverser le film complexe formé par la poche. Ainsi, avantageusement, il y a aussi moins de transfert fluidique de part et d'autre de la poche.

Toutefois, se limiter à ces faits intuitifs pour résoudre le problème technique susmentionné n'aurait pas été suffisant car n'importe quel fluide avec de grosses molécules ne peut pas être utilisé dans le fluide intermédiaire. En effet, il faut que le deuxième fluide soit compatible avec le premier fluide, mais également avec un fluide de stérilisation appliqué ultérieurement extérieurement sur le réservoir, d'où le choix d'un deuxième fluide qui soit également de traitement ophtalmique.

L'utilisation d'un deuxième fluide de traitement ophtalmique pour composer le fluide intermédiaire à injecter dans la cartouche est tout particulièrement inventif étant donné qu'en pratique, le premier fluide est typiquement mis sous pression dans la poche par de l'air comprimé, qui est facile à se procurer, à injecter et peu coûteux, ce qui n'est pas nécessairement le cas du deuxième fluide de traitement ophtalmique. L'usage de ce dernier est également particulier car son usage à des fins médicales est indirect, au sens où il n'est pas prévu que ce soit ce deuxième fluide de traitement ophtalmique qui soit pleinement utilisé pour un tel traitement.

Les sous-étapes (ii.1) et (ii.2) amplifient, quant à elles, l'effet technique induit par la présente invention. En effet, elles permettent d'accroître la proportion de deuxième fluide dans la cartouche, de façon à maximiser les effets techniques de l'invention. En effet, au moins d'air il y a dans la cartouche, autour de la poche, dans le réservoir, et au plus la proportion de fluide de traitement ophtalmique sera stable dans la poche au fil du temps. La sous-étape (ii.1) est tout particulièrement avantageuse dans ce cas car elle permet une extraction active de l'air « ambiant » naturellement présent dans la cartouche, et son remplacement par du deuxième fluide.

Dans le cadre de ce document, le terme « fluide de traitement ophtalmique » désigne tout fluide adapté pour être injecté au niveau de l'œil d'un patient en vue d'un traitement médical ophtalmologique. Un tel fluide ne consiste typiquement pas en de l'air. Les premier et deuxième fluides de traitement ophtalmologique (ci-après souvent nommé plus simplement « premier fluide » et « deuxième fluide ») sont généralement à l'état gazeux dans conditions de température et de pression normales pour l'utilisation du réservoir. De préférence, le premier et le deuxième fluides consistent en des gaz halogénés, préférentiellement des gaz perfluorés, par exemple, des perfluorocarbures.

Par exemple, dans le cas du traitement d'un décollement de la rétine par tamponnement, le premier fluide consiste préférentiellement en de l'hexafluorure de soufre (SF6), de l'hexafluoroéthane (C2F6) ou de l'octafluoropropane (C3F8), et le deuxième fluide consiste préférentiellement en un de ces trois gaz ou du tétraflurométhane (CF4).

De façon plus générale, et préférentiellement, les fluides mentionnées dans le cadre de ce document sont sous forme gazeuse, ou partiellement gazeuse.

L'homme du métier comprendra, par ailleurs, que l'invention peut aisément être exploitée avec d'autres types de fluides, dans d'autres domaines techniques (médicaux ou non) que l'ophtalmologie. Par exemple, les termes « de traitement ophtalmique » sont remplaçables par « de traitement médical » dans le cadre de ce document, partout où cela peut s'appliquer.

Dans le cadre de ce document, la cartouche consiste de préférence en un contenant creux rigide, apte à contenir la poche et le fluide intermédiaire. Elle peut être constituée de différent matériaux rigides comme de l'aluminium, de l'acier, du fer, ou du verre. L'usage d'aluminium généralement est préféré pour des raisons de coût (bon marché), de poids (léger), de résistance (par exemple, aux chocs) et/ou de facilité d'utilisation (maniable). La rigidité de la cartouche permet d'éviter toute contamination du fluide intermédiaire et/ou du premier fluide, en particulier lorsque, dans une étape ultérieure à sa fabrication, le réservoir est stérilisé par pulvérisation d'un fluide de stérilisation de façon externe, ce fluide de stérilisation comprenant typiquement de l'oxyde d'éthylène, et consistant de façon optionnelle en de l'oxyde d'éthylène (pur).

La rigidité de la cartouche permet également avantageusement d'optimiser le procédé selon l'invention tel qu'il sera décrit ci-après.

Dans le cadre de ce document, le terme « valve à poche » désigne, tel qu'il est bien connu par un homme du métier, une poche apte à contenir un fluide et portée par une valve. Dans le cas de l'invention, la valve est de préférence une valve étant rappelée élastiquement axialement vers l'extérieur (de la cartouche) en position d'obturation et pouvant être repoussée axialement vers l'intérieur (de la cartouche) en vue de son ouverture pour libérer du premier fluide. Cette valve permet également l'injection du premier fluide dans la poche en exerçant une pression (mécanique) de l'extérieur vers l'intérieur de la cartouche. Une valve semblable est notamment connue du brevet EP1589926B2.

Préférentiellement, la valve à poche comprend un manchon agencé autour de la valve et serti au niveau d'un épaulement externe par la paroi d'obturation. Un joint d'étanchéité est de préférence interposé entre l'extrémité du manchon et la paroi d'obturation.

La fixation de l'étape (iii) correspond de préférence à un sertissage, de sorte que le terme « fixer » correspond de préférence à « sertir » à l'étape (iii). Dans ce cas, l'étape (iii) se fait typiquement au moyen d'une dudgeonneuse tel que connu de l'homme du métier. L'invention n'est toutefois pas limitée à ce type de fixation de la paroi d'obturation sur la cartouche. Par exemple, une fixation par vissage ou par collage est également envisageable dans le cadre de l'invention.

Dans le cadre du présent document, l'usage des verbes « comprendre », « inclure », de leurs variantes, et de leurs conjugaisons, ne peut en aucune façon exclure la présence d'éléments autres que ceux mentionnés. De même, l'usage, dans ce document, de l'article indéfini « un », « une », ou de l'article défini « le », « la » ou « l' », pour introduire un élément n'exclut pas la présence d'une pluralité de ces éléments.

Dans le cadre de ce document, les valeurs de pression mentionnées sont toujours données en terme de pression absolue, c'est-à-dire de pression relative à la pression 0 dans l'espace vide.

Les termes « premier » et « deuxième » sont, quant à eux, utilisés dans ce document exclusivement pour différencier linguistiquement différents éléments, et ce sans impliquer d'ordre entre eux, ni impliquer que ces éléments présenteraient une différence structurelle ou de composition. En particulier, le cas où les premier et deuxième fluides sont essentiellement identiques ou identiques (ou, en d'autres termes s'ils sont identiques, sont un même fluide de traitement ophtalmologique), est parfaitement envisageable, et même préféré, dans le cadre de l'invention.

En effet, dans ce cas, lorsque du fluide intermédiaire pénètre dans la poche, la proportion en premier fluide en est sensiblement moins affectée, étant donné que le fluide intermédiaire comprend lui-même du premier fluide. En outre, l'usage du premier fluide comme deuxième fluide permet de satisfaire aux exigences de compatibilité entre ces deux fluides et un fluide de stérilisation éventuel comme il est introduit ci-dessus. Par cette réalisation préférée de l'invention, il est obtenu un réservoir dont le contenu de la poche est plus stable et plus pur en premier fluide, ce qui est d'autant plus important en vue de son dosage et de son administration ultérieure à un patient.

Dans le cadre de l'invention, les premier et deuxième fluides peuvent tout aussi bien être distincts.

Dans ce cas en particulier (mais bien évidemment aussi dans le cas où les premier et deuxième fluide sont identiques), le premier fluide a de préférence un taux d'expansion ophtalmique plus grand (>) ou égal (=) au deuxième fluide.

Dans le contexte technique de l'invention, tel qu'il est connu de l'homme du métier, le « taux d'expansion ophtalmique » est associé à l'expansion que peut subir une bulle du fluide, typiquement sous forme gazeuse, une fois injecté dans une cavité oculaire du patient. En particulier, dans le cas d'un tamponnement pour une chirurgie consécutive à un décollement de la rétine, du gaz fluoré, consistant en le premier fluide, une fois injecté dans la cavité oculaire du patient, forme une bulle qui se répand dans la cavité oculaire et génère une certaine pression intraoculaire afin de maintenir la rétine en place pendant sa cicatrisation. Le gaz se dissipe ensuite naturellement dans les 10 à 60 jours. Les gaz fluorés (ou plus précisément perfluorés) typiques utilisés dans ce contexte technique sont l'hexafluorure de soufre (SF6), l'hexafluoroéthane (C2F6) et l'octafluoropropane (C3F8), ceux-ci ayant des taux d'expansion ophtalmique respectifs d'environ 3, 3,3 et 4. Ces différents taux d'expansion ophtalmique correspondent à un facteur d'expansion d'une bulle de ces gaz fluorés purs injectés dans la cavité oculaire. Bien entendu, en pratique pour une telle intervention, le gaz fluoré est dosé, et en particulier dilué au moins en partie avec de l'air, préalablement à son injection. De façon générale, le taux d'expansion ophtalmique correspond à un rapport entre le volume d'une bulle de fluide (sans dilution) après injection et expansion dans la cavité oculaire et celui lors de son injection.

Le tétraflurométhane (CF4) n'est généralement pas utilisé comme premier fluide dans ce cadre car son tamponnement est moins efficace. Il peut toutefois être employé comme deuxième fluide dans ce contexte technique, quel que soit le premier fluide utilisé, et a un taux d'expansion ophtalmique plus petit que celui de l'hexafluorure de soufre.

L'usage d'un deuxième fluide au taux d'expansion ophtalmique au plus égal à celui du premier fluide est en particulier avantageux dans ce contexte. En effet, comme expliqué précédemment, lorsque le premier fluide est prélevé du réservoir dans la seringue du dispositif du brevet EP1589926B2, il est dosé pour produire un effet de tamponnement optimal. Ce dosage peut varier significativement selon la nature du premier fluide (par exemple, SF6, C2F6 ou C3F8). Si trop de premier fluide est injecté dans la cavité oculaire du patient, une pression intraoculaire trop élevée peut en résulter, et engendrer une cécité. En d'autres termes, si le dosage est inadaptée au taux d'expansion ophtalmique, la sécurité du patient n'est plus assurée.

Or, dans le cas où le fluide prélevé du réservoir comprend une quantité de deuxième fluide qui serait due à un échange fluidique via les parois de la poche, le taux d'expansion ophtalmique de ce fluide est susceptible de varier par rapport au taux d'expansion ophtalmique théorique du premier fluide. Ainsi, si le taux d'expansion ophtalmique du deuxième fluide est plus grand que celui du premier fluide, le taux d'expansion ophtalmique du fluide réellement prélevé du réservoir est susceptible d'être plus grand que celui du premier fluide, de telle sorte que l'application du dosage prévu pour le premier fluide deviendrait inadapté au taux d'expansion ophtalmique du fluide réellement prélevé du réservoir. Dans ce cas, l'injection du fluide prélevé du réservoir après un dosage prévu pour le premier fluide ferait courir les risques susdits au patient. Le choix d'un deuxième fluide au taux d'expansion ophtalmique inférieur ou égal à celui du premier fluide permet dès lors d'assurer la sécurité pleine et entière du patient lors de ces opérations.

Cet avantage est cependant limité lorsque le réservoir, et en particulier la poche, est sans défaut, car seule une quantité infime et négligeable de deuxième fluide est au final apte à pénétrer dans la poche pendant toute la durée de vie du réservoir (en général, trois à cinq ans) pour les raisons mentionnées ci-dessus. Cette quantité négligeable n'affecte donc essentiellement pas le dosage ultérieur, même si le deuxième fluide a un plus grand taux d'expansion ophtalmique que le premier fluide. Cette quantité est inférieure à 2%, de préférence à 1%, et de façon plus préférée à 0,5%, en volume de deuxième fluide présent dans le fluide prélevé du réservoir lorsque les premier et deuxième fluides sont distincts.

Cette réalisation joue un rôle plus important dans le cas extrêmement rare où la poche serait défectueuse. En effet, si une quantité significative de fluide intermédiaire pénétrerait dans la poche par un brèche de celle-ci, l'application du dosage ultérieur prévu pour le premier fluide au contenu de la poche (constitué du premier fluide et du fluide intermédiaire), bien qu'étant susceptible d'affecter le résultat final de l'intervention chirurgicale, ne fera courir aucun risque de surpression intraoculaire ou de cécité à l'œil du patient. C'est pour cette situation extrême et très rare que ce mode de réalisation s'avère réellement avantageux.

En bref, et selon un mode de réalisation préféré de l'invention, chacun des premier et deuxième fluides consiste en un gaz parmi : CF4, SF6, C2F6 ou C3F8. Plus préférentiellement :
- le premier fluide consiste en du gaz SF6, et le deuxième fluide consiste en du gaz CF4 ou SF6 ; ou
- le premier fluide consiste en du gaz C2F6, et le deuxième fluide consiste en du gaz CF4, SF6 ou C2F6 ; ou
- le premier fluide consiste en du gaz C3F8, et le deuxième fluide consiste en du gaz CF4, SF6, C2F6 ou C3F8.

Dans ce cas, de préférence, le deuxième fluide consiste en du gaz SF6 dans les trois cas, car ce gaz est à la fois un gaz de traitement ophtalmique classiquement utilisé pour le traitement d'un décollement de la rétine par tamponnement, et car il possède un plus faible taux d'expansion ophtalmique que les gaz C2F6 ou C3F8.

Selon un mode de réalisation possible de l'invention, l'étape (ii) comprend une injection de deuxième fluide et d'air dans la cartouche, au moins le deuxième fluide ou l'air étant au moins partiellement sous forme comprimée. Cette injection peut se faire simultanément ou successivement, dans n'importe quel ordre. Dans ce cas, fluide intermédiaire est alors de préférence constitué d'air et du deuxième fluide. Ce mode de réalisation est avantageusement simple à mettre en oeuvre et permet d'obtenir un réservoir proche en terme de contenu de l'art antérieur tout en présentant les avantages de l'invention.

Alternativement, selon un autre mode de réalisation préféré du procédé de l'invention, le fluide intermédiaire est constitué du deuxième fluide. Ce dernier est de préférence injecté totalement sous forme comprimée.

La pression dans la cartouche à l'issu de l'étape (ii) est de préférence entre 1,4 et 1,7 bar, de préférence d'environ 1,5 à 1,6 bar.

Avantageusement, ce mode de réalisation du procédé permet d'obtenir un réservoir dont tout l'espace dans la cartouche autour de la poche est rempli d'un fluide ambiant en surpression constitué d'air (qui était initialement présent dans la cartouche) et du fluide intermédiaire. Le fluide ambiant comprend généralement entre 35 et 70% en volume de deuxième fluide. La valeur de cette proportion est dépendante de la quantité et de la proportion de compression du deuxième fluide injecté. Toutefois, même lorsqu'elle est de 35%, cette proportion suffit à produire les effets techniques avantageux mentionnés en début d'exposé de l'invention, de façon particulièrement simple techniquement.

Selon l'invention, l'étape (ii) comprend les sous-étapes (ii.1) et (ii.2) comme susmentionnées. Elles sont de préférence exécutées dans cet ordre ou de façon simultanée. Bien que l'invention telle que présentée dans les revendications soit exposée avec ces sous-étapes (ii.1) et (ii.2), l'étendue de la présente divulgation n'est pas limitée à celles-ci et comprend notamment toute réalisation du procédé selon les étapes génériques (i) à (iv) exposées en deuxième phrase (ou alinéa) de la section d'exposé de l'invention. De manière générale, comme le comprendra un homme du métier à partir de la présente divulgation, l'un quelconque des modes de réalisation présentés à partir de ce procédé général peut être considéré seul ou en combinaison avec d'autres modes de réalisation, en particulier avec les sous-étapes (ii.1) et (ii.2). La dépendance des revendications peut en outre être considérée de manière potentiellement plus large de sorte que n'importe laquelle des combinaisons des revendications techniquement possibles et comprises par l'homme du métier, notamment au vu de la présente divulgation, fait partie de la présente divulgation.

Comme exposé précédemment, la réalisation de l'étape (ii) comprenant les sous-étapes (ii.1) et (ii.2) permet avantageusement d'accroître la proportion de deuxième fluide dans la cartouche, de façon à maximiser les effets techniques de l'invention, et en particulier lorsque le fluide intermédiaire est essentiellement (ou totalement) composé du deuxième fluide. La sous-étape (ii.1) d'extraction d'air de la cartouche peut se faire par différentes techniques aptes à induire l'aspiration par dépression de l'air présent dans la cartouche.

De préférence, cette dépression varie de préférence entre de 50 à 750 mbar (en pression absolue), et est, par exemple d'environ 100, 150, 200, 250, 300, 450 ou 500 mbar (en pression absolue), dans la cartouche. Cette valeur est à limiter afin de ne pas déformer la cartouche lors d'une exécution du procédé. Elle peut varier en particulier selon le matériau dont est constitué la cartouche. Ainsi, par exemple, la valeur de 150 mbar (en pression absolue) est tout à fait adaptée pour une cartouche faite d'aluminium (d'une épaisseur de paroi comprise, par exemple, entre 0,25 et 0,35 mm). Pour une cartouche faite de verre, l'aspiration induite par la dépression peut être accrue avec, par exemple, une dépression de l'ordre de 50 mbar (en pression absolue), de sorte qu'il est possible d'extraire davantage d'air à l'étape (ii.1). Il convient de noter de ces considérations que l'étape (ii.1) est en particulier rendue possible et aisé par le caractère rigide de la cartouche.

Selon un réalisation préféré du procédé selon l'invention, la séquence des sous-étapes (ii.1) et (ii.2) est répétée plusieurs fois à l'étape (ii). Les sous-étapes (ii.1) et (ii.2) sont plus préférentiellement répétées deux, trois, quatre ou cinq fois.

Avantageusement, grâce à ce mode de réalisation préféré, il est rendu possible d'accroître la proportion de deuxième fluide dans la cartouche par rapport à la réalisation du procédé sans cette répétition. En effet, comme noté ci-dessus, lors de la sous-étape (ii.1), l'extraction de l'air se fait dans les limites structurelles de la cartouche. La répétition de la sous-étape (ii.1) après la sous-étape (ii.2) permet de pallier à ces limites et par conséquent de remplacer plus d'air par du fluide intermédiaire que cela ne l'aurait été avec un seule occurrence de ces sous-étapes.

De façon plus précise, une fois le réservoir fabriqué, tout l'espace dans la cartouche, autour de la poche, est rempli par un fluide ambiant en suppression constitué d'air (par exemple, de l'air résiduel présent dans la cartouche qui n'a pu être extrait) et du fluide intermédiaire, de sorte que ce fluide ambiant comprend entre 35 et 95% en volume de deuxième fluide. Cette proportion en deuxième fluide est généralement de l'ordre de 70 à 80% en volume sans la répétition sous-étapes (ii.1) et (ii.2), et atteint de 80 à 95%, et même de 85 à 95%, avec cette répétition. Au plus cette proportion est grande, et au plus la quantité de fluide de traitement ophtalmique, et particulier la quantité de premier fluide si les premier et deuxième fluides sont identiques, dans la poche du réservoir reste stable au fil du temps.

Il a ainsi pu être expérimenté par l'inventeur que pour une telle proportion d'environ 90% en volume en premier fluide dans la cartouche, autour de la poche, si la poche comprend, après la fabrication du réservoir, environ 97% en volume de ce même premier fluide, trois ans plus tard, cette proportion restait d'environ 97%. De façon, plus générale, en répétant au moins une fois les sous-étapes (ii.1) et (ii.2), dans ce contexte technique, une proportion d'au moins 95% en volume du premier fluide est stable dans le temps à 1 % près pendant au moins trois ans.

Dans le cadre du présent document, les proportions de fluide de traitement ophtalmique fournie en % en volume peuvent être mesurées par chromatographie tel qu'il est connu de l'homme du métier. Une marge d'erreur de 5% est applicable à ces proportions étant donné que l'extraction du fluide permettant ces mesures induit généralement une pollution minime du fluide par de l'air. Afin de mesurer la proportion de premier fluide (et/ou en premier et deuxième fluides) contenu dans la poche du réservoir par rapport à l'ensemble du fluide délivrable contenu dans la poche, il peut être procédé, par exemple, à l'ouverture de la valve, au prélèvement d'une quantité de fluide délivrable dans une seringue, et à l'analyse de ce fluide par chromatographie pour déterminer la proportion en question.

Lors d'une répétition de la sous-étape (ii.1), l'extraction de l'air comprend en général aussi de l'extraction du deuxième fluide injecté dans la cartouche, étant donné qu'une séparation entre l'air et le deuxième fluide n'est en général pas faite lors de l'aspiration, notamment lorsque celle-ci est réalisé par dépression. Ceci n'empêche toutefois pas d'augmenter progressivement la proportion de deuxième fluide dans la cartouche à chaque répétition des sous-étapes (ii.1) et (ii.2). Il peut cependant être utile de ne pas injecter du deuxième fluide sous forme comprimée aux occurrences de la sous-étape (ii.2) précédant la dernière occurrence de sorte que la pression dans la cartouche soit élevée, et ce afin de limiter les pertes en deuxième fluide lors de l'occurrence suivante de l'étape (ii.1) suivante. Ainsi, cette pression est préférentiellement l'ordre de 1,2 bar pour les occurrences de la sous-étape (ii.2) précédant la dernière occurrence, et de l'ordre de 1,5 à 1,6 bar pour la dernière telle occurrence. En d'autres termes, le deuxième fluide est injecté dans la cartouche à la dernière occurrence de l'étape (ii.2) de préférence sous forme davantage comprimée qu'aux autres occurrences de l'étape (ii.2).

L'invention n'est bien sûr pas limitée à ces valeurs. En particulier, elles sont données à titre indicatif pour réaliser l'invention dans le cadre technique du brevet EP1589926B2, c'est-à-dire de telle façon que le premier fluide dans la poche soit en suppression dans le réservoir de sorte qu'une quantité adaptée de premier fluide soit libérée dans la seringue lors de l'ouverture de la valve. En particulier, dans ce cas, ces valeurs sont plutôt faibles. D'autres valeurs de pression plus ou moins grandes sont bien sûr tout à fait acceptables dans les limites structurelles de la cartouche tel qu'il sera compris aisément par l'homme du métier (par exemple, la pression interne à la cartouche peut atteindre au plus 5 à 6 bar dans une cartouche de 10 à 100 ml, par exemple, de 12, 30 ou 75 ml, en aluminium, par exemple d'une épaisseur de paroi de 0,25 à 0,35 mm, ce type de cartouche étant typiquement utilisée dans le cadre de l'invention).

De façon générale et préférée, l'étape (ii), et préférentiellement l'étape (iii), sont réalisées au sein de l'enceinte close. Cette enceinte permet notamment, lors de l'étape (ii), une gestion adéquate de la diminution de la proportion d'air dans la cartouche, ainsi qu'une implémentation adaptée des sous-étapes (ii.1) et (ii.2) sans contamination fluidique externe.

Selon un mode de réalisation préféré de l'invention, les étapes (ii) et (iii) sont plus particulièrement réalisées au sein d'une dudgeonneuse. L'enceinte au sein de laquelle sont placés la cartouche et la valve à poche à l'étape (i) fait alors préférentiellement partie de la dudgeonneuse.

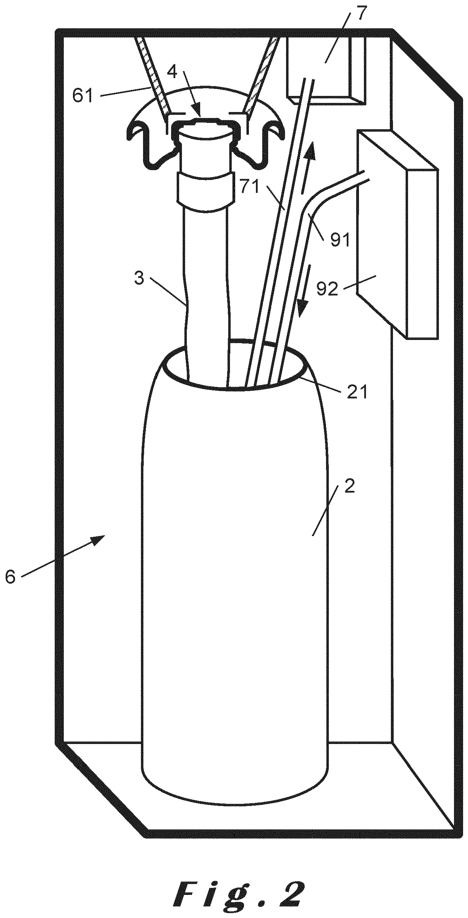

La dudgeonneuse est ainsi configurée pour exécuter pleinement l'étape (ii), en plus de l'étape (iii) de dudgeonnage, par exemple sur base d'une configuration telle que schématiquement illustrée en figure 2 ci-après introduite. De cette façon, ces étapes clés du procédé selon l'invention peuvent être exécutées facilement au sein d'un même outil, et d'une même enceinte, et de façon à garantir la bonne exécution du procédé. De préférence, la dudgeonneuse comprend une interface permettant à un utilisateur d'introduire des paramètres d'exécution du procédé tels que le nombre de répétition des étapes (ii.1) et (ii.2) lorsqu'il en comprend, les quantité de fluide intermédiaire et/ou de deuxième fluide à injecter, leur pression et/ou une pression intermédiaire ou finale au sein de la cartouche, ...

L'invention propose aussi un réservoir de fluide de traitement ophtalmique, et en particulier, un tel réservoir obtenu par le procédé selon l'invention.

En effet, un autre objet de l'invention est de fournir un tel réservoir qui soit davantage fiable que les réservoirs de l'art antérieur.

À cet effet, l'invention propose un réservoir d'un premier fluide de traitement ophtalmique comprenant :
- une cartouche rigide comprenant une ouverture,
- une valve à poche comprenant une poche portée par une valve prolongée d'une paroi d'obturation,
   la paroi d'obturation étant fixée sur la cartouche, autour de l'ouverture, de façon hermétique, la poche étant enfermée dans la cartouche,

dans lequel un fluide intermédiaire, au moins partiellement sous forme comprimée, est présent dans la cartouche autour de la poche,
dans lequel le premier fluide est maintenu sous pression dans la poche par le fluide intermédiaire présent dans la cartouche,
et dans lequel le fluide intermédiaire comprend un deuxième fluide de traitement ophtalmique.

Comme exposé ci-dessus, grâce aux sous-étapes (ii.1) et (ii.2), le réservoir est tel qu'un espace dans la cartouche autour de la poche est rempli (uniquement) par un fluide ambiant constitué d'air et de fluide intermédiaire, et ce fluide ambiant comprend entre 70 et 95% en volume de deuxième fluide.

Ce réservoir est plus fiable aux fins d'applications telles que celle du brevet EP1589926B2, et en particulier, le contenu de la poche est davantage stable au fil du temps, et ce pour les mêmes raisons qu'exposées ci-dessus dans l'exposé de l'invention. En particulier, tous les modes de réalisation et avantages du procédé selon l'invention exposés ci-dessus se transposent directement, *mutatis mutandis,* à ce réservoir.

Ainsi, par exemple, et de façon non exhaustive, selon différentes réalisations préférées compatible du réservoir introduites ci-dessous dans l'exposé :
- les premier et deuxième fluides sont (au moins) essentiellement identiques ; et/ou
- chacun des premier et deuxième fluide est choisi parmi un des gaz suivants : CF4, SF6, C2F6 ou C3F8 ; et/ou
- le premier fluide a un taux d'expansion ophtalmique plus grand ou égal que le deuxième fluide ; et/ou
- le fluide ambiant comprend génériquement entre 35 et 95% en volume, et de préférence entre 80 et 95% en volume, de deuxième fluide ; et/ou
- la poche est remplie (uniquement) d'un fluide délivrable contenant au moins une proportion de 95% en volume de premier fluide,
   et plus préférentiellement, si les premier et deuxième fluides sont identiques, la proportion de premier fluide est stable dans le temps à 1 % près pendant au moins trois ans.

Les considérations exposées ci-dessus sur la valve à poche et la cartouche s'étendent bien entendu aussi au présent réservoir selon l'invention. En outre, les considérations techniques exposées dans le brevet EP1589926B2 concernant le réservoir, et particulièrement la valve à poche et la cartouche, peuvent également s'appliquer par référence à l'invention.

Dans cet esprit, la présente invention propose un dispositif de préparation extemporanée d'une quantité de premier fluide de traitement ophtalmique en vue de son administration à un patient, comprenant un réservoir selon l'invention.

Le dispositif comprend de préférence une seringue ayant une extrémité de distribution prévue pour être reliée hermétiquement en communication fluidique avec la valve en vue de libérer du premier fluide de la poche dans la seringue. La seringue comprend de préférence un piston coulissant dans un cylindre tel qu'il est connu de l'homme du métier. La quantité de fluide délivrable (dont fait partie le premier fluide) contenu dans la poche et la quantité de fluide ambiant (dont fait partie le fluide intermédiaire, et particulièrement le deuxième fluide), sont adaptées pour qu'une quantité adaptée de premier fluide soit libérée dans la seringue lors d'une ouverture de la valve comme il est enseigné par le brevet EP1589926B2.

Le dispositif comprend de préférence également un élément de liaison et de stérilisation comprenant une partie pour stériliser par filtration du fluide délivrable s'écoulant d'une entrée et d'une sortie de cette partie. L'élément de liaison est de préférence configuré pour s'assembler hermétiquement avec, d'une part, la valve, et d'autre part, l'extrémité de distribution de la seringue, de façon à permettre le passage du fluide délivrable de la poche vers (le cylindre de) la seringue.

D'une façon générale, l'homme du métier comprendra que les modes de réalisation et les avantages décrits dans le brevet EP1589926B2 s'étendent à ce dispositif.

### Brève description des figures

D'autres caractéristiques et avantages de la présente invention apparaîtront à la lecture de la description détaillée qui suit pour la compréhension de laquelle on se reportera aux figures annexées parmi lesquelles :
- la figure 1 illustre une vue schématique en coupe d'un réservoir selon un mode de réalisation préféré de l'invention ;
- la figure 2 illustre une vue purement schématique tridimensionnelle d'une étape du procédé de fabrication du réservoir illustré en figure 1, selon l'invention.

Les dessins des figures ne sont pas à l'échelle. Des éléments semblables sont en général dénotés par des références semblables dans les figures. Dans le cadre du présent document, les éléments identiques ou analogues peuvent porter les mêmes références. En outre, la présence de numéros ou lettres de référence aux dessins ne peut être considérée comme limitative, y compris lorsque ces numéros ou lettres sont indiqués dans les revendications.

### Description détaillée de modes de réalisation de l'invention

Cette partie présente une description de modes de réalisation particuliers de l'invention en références à des figures. L'invention n'est cependant pas limitée par celles-ci. Les figures décrites ci-dessous ne sont que schématiques et ne sont pas limitantes.

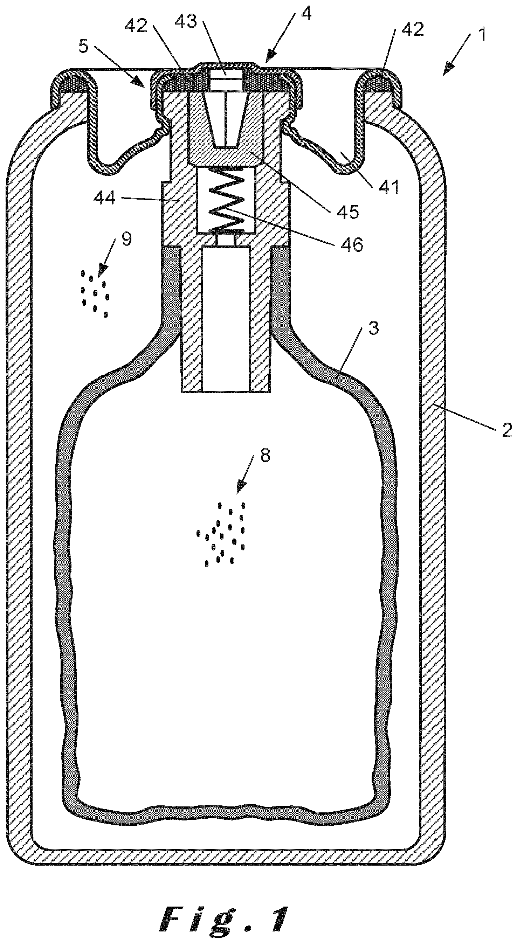

La figure 1 illustre un réservoir 1 selon un mode de réalisation préféré de la présente invention. Le réservoir 1 comprend une cartouche 2 rigide métallique qui forme un contenant pour fluides, et une valve à poche comprenant une poche 3 portée par une valve 4 qui est prolongée d'une paroi d'obturation 41. La poche 3 est enfermée dans la cartouche 2. La valve à poche est munie d'un manchon 44 agencé autour de la valve 4 et serti au niveau d'une portion de sertissage 5 par la paroi d'obturation 41. La paroi d'obturation 41 est en outre fixée, plus précisément sertie, sur la cartouche 2 par dudgeonnage, autour d'une ouverture 21 supérieure de la cartouche 2 (visible en figure 2), de façon hermétique. Comme il est connu d'un homme du métier du dudgeonnage, dans ce cas, la paroi d'obturation 41 est repoussée sous une zone de fixation de la cartouche 2. Pour obtenir un réservoir 1 hermétique, il est prévu d'interposé des joints d'étanchéité 42 entre la paroi d'obturation 41, et d'une part, le manchon 44, et d'autre part, la cartouche 2. Le manchon 44 comprend en outre une cavité enfermant un ressort de compression 46, et une soupape 45 qui est rappelée de façon élastique contre le joint d'étanchéité 42 agencé au niveau de la portion de sertissage 5, formant ainsi la valve 4. Celle-ci est dès lors rappelée élastiquement vers l'extérieur de la cartouche 2 en position d'obturation et peut être repoussée vers l'intérieur de la cartouche 2 pour libérer un fluide enfermé dans la poche 3 via une orifice 43 de distribution de la valve 4.

La cartouche 2 et la valve à poche telles que décrites structurellement ci-dessus peuvent correspondre à la cartouche et la valve à poche utilisée pour le réservoir du dispositif du brevet EP1589926B2.

La poche 3 est remplie d'un fluide délivrable par le réservoir 1 et constitué essentiellement d'un premier fluide 8 de traitement ophtalmique. Un autre fluide tel que de l'air est généralement présent en quantité résiduelle dans la poche 3 avant son remplissage, de sorte que le fluide délivrable en contient une quantité minime, généralement, de l'ordre de moins de 5% en volume, de préférence de l'ordre de moins de 3% en volume.

Le fluide délivrable, et en particulier le premier fluide 8, est maintenu sous pression dans la poche 3 par un fluide intermédiaire présent dans la cartouche 2, autour de la poche 3, sous une forme au moins partiellement comprimée. En pratique, tout l'espace à l'intérieur de la cartouche 2, autour de la poche 3, est rempli d'un fluide ambiant qui comprend, outre le fluide intermédiaire, un autre fluide tel que de l'air qui est naturellement présent dans la cartouche 2 avant qu'elle soit utilisée pour fabriquer le réservoir 1.

Comme il est expliqué dans l'exposé de l'invention, et commenté ci-après au regard de la figure 2, il est possible d'extraire au moins en partie cet air de la cartouche 2 lors du procédé de fabrication du réservoir 1, de façon à accroître la proportion de fluide intermédiaire dans le fluide ambiant.

Le fluide intermédiaire présent dans la cartouche 2 comprend un deuxième fluide 9 de traitement ophtalmique, et de préférence au moins 10% en volume de deuxième fluide 9. Selon une réalisation particulière et préférée du réservoir 1, le fluide intermédiaire consiste uniquement en ce deuxième fluide 9, celui-ci étant de préférence pressurisé, de sorte que le fluide ambiant comprend entre 35 et 95% en volume de deuxième fluide 9, et préférentiellement au moins 90% en volume de deuxième fluide 9 dans le cas où l'air présent initialement dans la cartouche 2 a pu être extrait de façon optimale. Le cas où le premier 8 et le deuxième 9 fluides ont la même constitution physico-chimique est préféré.

Le réservoir 1 est de préférence fabriqué selon le procédé de l'invention. La figure 2 illustre de façon purement schématique l'étape (ii) de ce procédé.

Dans cette étape, la cartouche 2 et la valve à poche sont placées dans une enceinte 6 close, préférentiellement l'enceinte d'une dudgeonneuse adaptées pour exécutée aussi l'étape (iii). La valve à poche, qui n'est pas encore fixée à la cartouche 2 à ce stade du procédé, est maintenue au moins en partie au-dessus de la cartouche 2 par des moyens de maintien 61, par exemple, au moins un joint, et/ou des pinces, et/ou des attaches. L'ouverture 21 de la cartouche est au moins partiellement libre pour accueillir des moyens de connexions étanches 71, 91, par exemple, des tuyaux ou autre moyens connus de l'homme du métier, pour l'extraction et l'injection de fluides au sein de la cartouche 2.

Par exemple, suivant une implémentation de la sous-étape (ii.1) d'un mode de réalisation du procédé, des moyens d'aspiration 7, par exemple sous la forme d'une source de vide d'air, induisent l'aspiration par dépression de l'air présent dans la cartouche 2 via la connexion étanche 71. Suivant une implémentation de la sous-étape (ii.2) de ce mode de réalisation du procédé, des moyens d'injection 92, par exemple sous la forme d'une bombonne de deuxième fluide 9 pressurisé, induisent une injection du deuxième fluide 9 dans la cartouche 2 via la connexion étanche 91. Tel qu'il sera aisément compris de l'homme du métier, ces opérations sont effectuées de façon étanche, dans le cadre hermétique de l'enceinte 6, pour garantir leur efficacité. Par exemple, une tête (non représentée) dans l'enceinte 6 est en outre de préférence agencée hermétiquement au niveau de l'ouverture 21 et traversée hermétiquement par les connexions 71, 91, de façon permettre une implémentation efficace de ces opérations successives d'extraction et d'injection.

En bref, l'invention concerne un procédé de fabrication d'un réservoir 1 d'un premier fluide 8 de traitement ophtalmique par injection d'un deuxième fluide 9 de traitement ophtalmique dans une cartouche 2, fixation d'une valve 4 portant une poche 3 sur la cartouche 2 et remplissage de la poche 3 en du premier fluide 8. L'invention concerne également un tel réservoir 1.

Cette invention a présentement été décrite en relation avec des modes de réalisations spécifiques, qui ont une valeur purement illustrative et ne doivent pas être considérés comme limitatifs. De manière générale, il apparaîtra évident pour l'homme du métier que l'invention n'est aucunement limitée aux exemples illustrés et/ou décrits ci-dessus.

## Revendications

1. Procédé de fabrication d'un réservoir (1) d'un premier fluide (8) de traitement ophtalmique comprenant les étapes suivantes :
(i) fournir et placer dans une enceinte (6) close :
- une cartouche (2) rigide comprenant une ouverture (21),
- une valve à poche comprenant une poche (3) portée par une valve (4) prolongée d'une paroi d'obturation (41),
(ii) injecter un fluide intermédiaire sous forme comprimée dans la cartouche (2) via l'ouverture (41),
le fluide intermédiaire comprenant un deuxième fluide (9) de traitement ophtalmique ;
(iii) fixer la paroi d'obturation (41) sur la cartouche (2), au niveau de l'ouverture (21), de façon hermétique, et de sorte que la poche (3) soit enfermée dans la cartouche (2) ;
(iv) injecter le premier fluide (8) dans la poche (3) via la valve (4), de sorte qu'il soit maintenu sous pression dans la poche (3) par le fluide intermédiaire présent dans la cartouche (2) autour de la poche (3) ;
**caractérisé en ce que** l'étape (ii) comprend les sous-étapes suivantes :
(ii.1) extraire de l'air de la cartouche (2) ;
(ii.2) injecter le deuxième fluide (9) sous forme comprimée dans la cartouche (2).

2. Procédé selon la revendication 1, dans lequel les premier (8) et deuxième (9) fluides sont essentiellement identiques.

3. Procédé selon la revendication 1, dans lequel le premier fluide (8) a un taux d'expansion ophtalmique plus grand que le deuxième fluide (9).

4. Procédé selon l'une quelconque des revendications précédentes, dans lequel chacun des premier (8) et deuxième (9) fluides consiste en un gaz parmi du tétraflurométhane (CF4), de l'hexafluorure de soufre (SF6), de l'hexafluoroéthane (C2F6) ou de l'octafluoropropane (C3F8).

5. Procédé selon l'une quelconque des revendications précédentes, dans lequel le fluide intermédiaire est constitué du deuxième fluide (9).

6. Procédé selon l'une quelconque des revendications précédentes, dans lequel la séquence des sous-étapes (ii.1) et (ii.2) est répétée plusieurs fois à l'étape (ii).

7. Procédé selon la revendication 6, dans lequel le deuxième fluide (9) est injecté dans la cartouche (2) à la dernière occurrence de l'étape (ii.2) sous forme davantage comprimée qu'aux autres occurrences de l'étape (ii.2).

8. Procédé selon l'une quelconque des revendications précédentes, dans les étapes (ii) et (iii) sont réalisées au sein d'une dudgeonneuse, l'enceinte (6) faisant partie de la dudgeonneuse.

9. Réservoir (1) d'un premier fluide (8) de traitement ophtalmique comprenant :
- une cartouche (2) rigide comprenant une ouverture (21),
- une valve à poche comprenant une poche (3) portée par une valve (4) prolongée d'une paroi d'obturation (41),
la paroi d'obturation (41) étant fixée sur la cartouche (2), autour de l'ouverture (21), de façon hermétique,
la poche (3) étant enfermée dans la cartouche (2),
dans lequel un fluide intermédiaire (9), sous forme comprimée, et comprenant un deuxième fluide (9) de traitement ophtalmique, est présent dans la cartouche (2) autour de la poche (3),
dans lequel le premier fluide (8) est maintenu sous pression dans la poche (3) par le fluide intermédiaire (9) présent dans la cartouche (2),
et dans lequel un espace dans la cartouche (2), autour de la poche (3), est rempli uniquement par un fluide ambiant constitué d'air et de fluide intermédiaire,
**caractérisé en ce que** le fluide ambiant comprend entre 70 et 95% en volume de deuxième fluide (9).

10. Réservoir (1) selon la revendication 9, dans lequel le fluide ambiant comprend entre 80 et 95% en volume de deuxième fluide (9).

11. Réservoir (1) selon la revendication 9 ou 10, dans lequel la poche (3) est remplie uniquement d'un fluide délivrable contenant au moins une proportion de 95% en volume de premier fluide (8).

12. Dispositif de préparation extemporanée d'une quantité de premier fluide (8) de traitement ophtalmique en vue de son administration à un patient, comprenant un réservoir (1) selon l'une quelconque des revendications 9 à 11 et une seringue ayant une extrémité de distribution adaptée pour être reliée hermétiquement et en communication fluidique avec la valve (4) en vue de libérer du premier fluide (8) de la poche (3) dans la seringue.

## Patentansprüche

1. Verfahren zur Herstellung eines Reservoirs (1) für ein erstes ophthalmisches Behandlungsfluid (8), das die folgenden Schritte umfasst:
(i) Bereitstellen und Platzieren in einer geschlossenen Kammer (6):
- einer starren Kartusche (2), die eine Öffnung (21) umfasst,
- eines Beutelventils, das einen Beutel (3) umfasst, der von einem Ventil (4) getragen wird, das von einer Verschlusswand (41) verlängert wird,
(ii) Injizieren eines Zwischenfluids in komprimierter Form über die Öffnung (41) in die Kartusche (2),
wobei das Zwischenfluid ein zweites ophthalmisches Behandlungsfluid (9) umfasst;
(iii) hermetisch dichtes Befestigen der Verschlusswand (41) im Bereich der Öffnung (21) an der Kartusche (2) und so, dass der Beutel (3) in der Kartusche (2) eingeschlossen ist;
(iv) Injizieren des ersten Fluids (8) über das Ventil (4) in den Beutel (3) so, dass es in dem Beutel (3) durch das in der Kartusche (2) um den Beutel (3) herum vorhandene Zwischenfluid unter Druck gehalten wird;
**dadurch gekennzeichnet, dass** Schritt (ii) die folgenden Teilschritte umfasst:
(ii.1) Extrahieren von Luft aus der Kartusche (2);
(ii.2) Injizieren des zweiten Fluids (9) in komprimierter Form in die Kartusche (2).

2. Verfahren nach Anspruch 1, wobei das erste (8) und das zweite (9) Fluid im Wesentlichen identisch sind.

3. Verfahren nach Anspruch 1, wobei das erste Fluid (8) eine größere ophthalmische Expansionsrate aufweist als das zweite Fluid (9).

4. Verfahren nach einem der vorstehenden Ansprüche, wobei jedes des ersten (8) und des zweiten (9) Fluids aus einem Gas aus Tetrafluormethan (CF4), Schwefelhexafluorid (SF6), Hexafluorethan (C2F6) oder Octafluorpropan (C3F8) besteht.

5. Verfahren nach einem der vorstehenden Ansprüche, wobei das Zwischenfluid aus dem zweiten Fluid (9) besteht.

6. Verfahren nach einem der vorstehenden Ansprüche, wobei die Abfolge der Teilschritte (ii.1) und (ii.2) in Schritt (ii) mehrmals wiederholt wird.

7. Verfahren nach Anspruch 6, wobei das zweite Fluid (9) beim letzten Vorkommen von Schritt (ii.2) in stärker komprimierter Form als bei den anderen Vorkommen von Schritt (ii.2) in die Kartusche (2) injiziert wird.

8. Verfahren nach einem der vorstehenden Ansprüche, wobei die Schritte (ii) und (iii) in einer Rohraufweitungseinrichtung ausgeführt werden, wobei die Kammer (6) Teil der Rohraufweitungseinrichtung ist.

9. Reservoir (1) für ein erstes ophthalmisches Behandlungsfluid (8), umfassend:
- eine starre Kartusche (2), die eine Öffnung (21) umfasst,
- ein Beutelventil, das einen Beutel (3) umfasst, der von einem Ventil (4) getragen wird, das von einer Verschlusswand (41) verlängert wird,
wobei die Verschlusswand (41) um die Öffnung (21) herum hermetisch dicht an der Kartusche (2) befestigt ist,
wobei der Beutel (3) in der Kartusche (2) eingeschlossen ist,
wobei ein Zwischenfluid (9), das in komprimierter Form vorliegt und ein zweites ophthalmisches Behandlungsfluid (9) umfasst, in der Kartusche (2) um den Beutel (3) herum vorhanden ist,
wobei das erste Fluid (8) im Beutel (3) durch das in der Kartusche (2) vorhandene Zwischenfluid (9) unter Druck gehalten wird,
und wobei ein Raum in der Kartusche (2) um den Beutel (3) herum nur mit einem Umgebungsfluid gefüllt ist, das aus Luft und Zwischenfluid besteht,
**dadurch gekennzeichnet, dass** das Umgebungsfluid zwischen 70 und 95 Vol.% zweites Fluid (9) umfasst.

10. Reservoir (1) nach Anspruch 9, wobei das Umgebungsfluid zwischen 80 und 95 Vol.-% zweites Fluid (9) umfasst.

11. Reservoir (1) nach Anspruch 9 oder 10, wobei der Beutel (3) nur mit einem abgabefähigen Fluid gefüllt ist, das mindestens einen Anteil von 95 Vol.-% erstes Fluid (8) enthält.

12. Vorrichtung zur extemporierten Zubereitung einer Menge eines ersten ophthalmischen Behandlungsfluids (8) im Hinblick auf dessen Verabreichung an einen Patienten, die ein Reservoir (1) nach einem der Ansprüche 9 bis 11 und eine Spritze umfasst, die ein Abgabeende aufweist, das geeignet ist im Hinblick darauf, erstes Fluid (8) aus dem Beutel (3) in die Spritze freizugeben, hermetisch dicht und in strömungstechnischer Kommunikation mit dem Ventil (4) verbunden zu werden.

## Claims

1. A method for manufacturing a reservoir (1) of a first ophthalmic treatment fluid (8) comprising the following steps:
(i) supplying and placing in a closed enclosure (6):
- a rigid cartridge (2) comprising an opening (21),
- a pouch valve comprising a pouch (3) carried by a valve (4) extended by a closure wall (41),
(ii) injecting an intermediate fluid in compressed form into the cartridge (2) via the opening (41),
the intermediate fluid comprising a second ophthalmic treatment fluid (9);
(iii) attaching the closure wall (41) to the cartridge (2), at the level of the opening (21), in a hermetically sealed manner, so that the pouch (3) is enclosed in the cartridge (2);
(iv) injecting the first fluid (8) into the pouch (3) via the valve (4),
so that it is maintained under pressure in the pouch (3) by the intermediate fluid present in the cartridge (2) around the pouch (3);
**characterized in that** step (ii) comprises the following sub-steps:
(ii.1) extracting air from the cartridge (2);
(ii.2) injecting the second fluid (9) in compressed form into the cartridge (2).

2. The method according to claim 1, wherein the first (8) and second (9) fluids are essentially identical.

3. The method according to claim 1, wherein the first fluid (8) has a greater ophthalmic expansion rate than the second fluid (9).

4. The method according to any one of the preceding claims, wherein each of the first (8) and second (9) fluids consists of a gas from among tetrafluromethane (CF4), sulphur hexafluoride (SF6), hexafluoroethane (C2F6) or octafluoropropane (C3F8).

5. The method according to any one of the preceding claims, wherein the intermediate fluid consists of the second fluid (9).

6. The method according to any one of the preceding claims, wherein the sequence of sub-steps (ii.1) and (ii.2) is repeated several times in step (ii).

7. The method according to claim 6, wherein the second fluid (9) is injected into the cartridge (2) at the last occurrence of step (ii.2) in a more compressed form than at the other occurrences of step (ii.2).

8. The method according to any one of the preceding claims, wherein steps (ii) and (iii) are carried out within a tube expander, the enclosure (6) forming part of the tube expander.

9. A reservoir (1) for a first ophthalmic treatment fluid (8) comprising:
- a rigid cartridge (2) comprising an opening (21),
- a pouch valve comprising a pouch (3) carried by a valve (4) extended by a closure wall (41),
the closure wall (41) being hermetically sealed to the cartridge (2) around the opening (21),
the pouch (3) being enclosed in the cartridge (2),
wherein an intermediate fluid (9), in compressed form, and comprising a second ophthalmic treatment fluid (9), is present in the cartridge (2) around the pouch (3), wherein the first fluid (8) is maintained under pressure in the pouch (3) by the intermediate fluid (9) present in the cartridge (2),
and wherein a space in the cartridge (2), around the pouch (3), is filled solely with an ambient fluid consisting of air and intermediate fluid,
**characterized in that** the ambient fluid comprises between 70 and 95% by volume of second fluid (9).

10. The reservoir (1) according to claim 9, wherein the ambient fluid comprises between 80 and 95% by volume of second fluid (9).

11. The reservoir (1) according to claim 9 or 10, wherein the pouch (3) is filled solely with a dispensable fluid containing at least 95% by volume of a first fluid (8).

12. A device for the extemporaneous preparation of a quantity of first fluid (8) for ophthalmic treatment with a view to its administration to a patient, comprising a reservoir (1) according to any one of claims 9 to 11 and a syringe having a dispensing end adapted to be connected hermetically and in fluid communication with the valve (4) with a view to release the first fluid (8) from the pouch (3) into the syringe.
